(19) Europäisches Patentamt / European Patent Office / Office européen des brevets

(11) **EP 4 361 626 B1**

(12) **FASCICULE DE BREVET EUROPEEN**

(45) Date de publication et mention
de la délivrance du brevet:
**15.04.2026 Bulletin 2026/16**

(21) Numéro de dépôt: **23206320.6**

(22) Date de dépôt: **27.10.2023**

(51) Classification Internationale des Brevets (IPC):
**G01N 29/04** *(2006.01)* **G01N 29/44** *(2006.01)*

(52) Classification Coopérative des Brevets (CPC):
**G01N 29/04; G01N 29/4427**

(54) **PROCÉDÉ DE CONTRÔLE NON DESTRUCTIF D'UNE PLURALITÉ D'ÉLÉMENTS**

VERFAHREN ZUR ZERSTÖRUNGSFREIEN PRÜFUNG EINER VIELZAHL VON ELEMENTEN

METHOD FOR NON-DESTRUCTIVE TESTING OF A PLURALITY OF ELEMENTS

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **28.10.2022 FR 2211287**

(43) Date de publication de la demande:
**01.05.2024 Bulletin 2024/18**

(73) Titulaire: **Electricité de France**
**75008 Paris (FR)**

(72) Inventeurs:
• **PAUL, Nicolas**
**93100 Montreuil (FR)**
• **COURET, Laura**
**92300 Levallois-Perret (FR)**

(74) Mandataire: **Regimbeau**
**20, rue de Chazelles**
**75847 Paris Cedex 17 (FR)**

(56) Documents cités:
**US-A1- 2022 152 628**

• **HENDRICKX KILIAN ET AL: "A general anomaly detection framework for fleet-based condition monitoring of machines", MECHANICAL SYSTEMS AND SIGNAL PROCESSING, ELSEVIER, AMSTERDAM, NL, vol. 139, 11 January 2020 (2020-01-11), XP086063635, ISSN: 0888-3270, [retrieved on 20200111], DOI: 10.1016/ J.YMSSP.2019.106585**

**Description**

**Domaine technique**

L'invention appartient au domaine du contrôle non destructif d'une pluralité d'éléments, afin de déterminer un caractère sain ou non-sain de l'élément. Plus précisément, l'invention s'applique au contrôle d'éléments similaires à partir de signaux de mesure, chacun des signaux de mesure correspondant à un élément différent déterminé.

**Arrière-plan technologique**

Les procédés de contrôle non destructif reposent sur la recherche d'anomalies dans des signaux de mesure, susceptible de correspondre à un défaut dans un élément non sain. Les signaux de mesure peuvent être de différentes natures, et par exemple sont des signaux résultant d'acquisitions ultrasonores, ou des réponses électriques à stimulations telles que la génération de courant de Foucault, la détection d'ondes radio, ou bien encore des images ou vidéos.

Dans ces signaux de mesure, la recherche d'anomalies peut être rendue difficile. En effet, de multiples facteurs sont susceptibles d'altérer les signaux de mesure, comme par exemple une géométrie complexe de l'élément inspecté ou un défaut de propreté de celui-ci, et la présence d'une anomalie peut être cachée parmi ces altérations. En outre, si la détection doit être faite par un opérateur humain analysant les signaux de mesure, le contrôle peut être très long et fastidieux, et sa qualité dépend de la compétence de cet opérateur. Ces problèmes peuvent devenir très gênants dans des domaines où le nombre d'éléments à inspecter est important et/ou quand il est primordial de pouvoir détecter tous les défauts pour des raisons de sûreté.

Il a été proposé diverses méthodes permettant la recherche d'anomalies susceptibles de correspondre à des défauts dans des signaux de mesure. La plupart de ces méthodes reposent sur la comparaison de valeurs dérivées des signaux avec des référence. Cependant, chaque méthode est alors spécifique à une application particulière, et la sensibilité de la méthode dépend beaucoup de la pertinence du calcul des valeurs dérivées et de la définition de la référence utilisée. Ces méthodes nécessitent donc généralement une longue mise au point et peuvent ne pas être adaptées pour détecter des défauts non anticipés ou pour inspecter d'autres éléments que celle pour laquelle elle a été conçue.

Par exemple, la demande de brevet FR3029288 propose une inspection des pénétrations de fond de cuve ; pour chaque échantillon temporel, des valeurs statistiques telles que la moyenne ou l'écart-type des valeurs prises par le signal à cet instant sont calculées sur les P signaux disponibles. Ces statistiques sont ensuite utilisées pour construire un signal homogénéisé temporellement, avec, pour chaque échantillon temporel, une moyenne constante égale à zéro et un écart-type constant égal à 1. Si l'application de ce procédé à des pénétrations de fond de cuve donne des résultats satisfaisants, elle n'est pas aussi efficace lorsqu'elle est appliquée à l'inspection d'autres éléments tels que des vis de cloisonnement. En effet, les défauts ne génèrent pas systématiquement des augmentations d'amplitudes, mais souvent des variations de forme des signaux. D'autre part, les signaux ne sont pas exactement synchronisés les uns par rapport aux autres, des dilatations/contractions/décalages locaux peuvent apparaître.

Van Vaerenbergh et al. "Pattern Localization in Time Series Through Signal-To-Model Alignment in Latent Space" February 2018, DOI:10.1109/ICASSP.2018.8461890, Conference: ICASSP 2018 - IEEE International Conference on Acoustics, Speech and Signal Processing, Calgary, Alberta, Canada" décrit un procédé de localisation une séquence de motifs prédéfinie dans une série temporelle de test non-destructif, basé sur un apprentissage automatique pour accroître une similarité entre une série temporelle synthétisée et une série temporelle réelle.

Hendrickx K et al, "A general anomaly detection framework for fleet-based condition monitoring of machines", Mecahnical Systems and Signal Processing, Elsevier, Amsterdam, vol. 139, 11 janvier 2020, décrit un procédé de détection d'anomalies de machines pour la surveillance d'une flotte de machines similaires. Des paires de machines sont formées et des signaux de mesure, chacun correspondant à une machine, sont comparés et analysés statistiquement, en particulier à l'aide de la considération d'une déformation temporelle dynamique, ou DTW pour l'anglais « Dynamic Time Warping ».

**Présentation de l'invention**

L'invention vise donc à proposer un procédé de contrôle non destructif d'une pluralité d'éléments à partir de signaux de mesure, qui soit simple à mettre en œuvre, applicable au contrôle d'un grand nombre d'éléments similaires, et qui permette de détecter les défauts de manière fiable.

Il est proposé un procédé de contrôle non destructif d'une pluralité d'éléments similaires à partir de signaux de mesure, chacun des signaux de mesure correspondant à un élément différent déterminé, comprenant les étapes suivantes :

- obtention d'une suite d'au moins une portion de signal à partir de chaque signal de mesure,
- pour chaque couple associant deux portions de signal présentant une même position dans leur suite respective, une distance entre ces portions de signal dudit couple est calculée, ladite distance étant calculée en utilisant une déformation temporelle dynamique,
- pour chaque portion de signal, détermination d'au moins un indicateur statistique à partir des distances de couples impliquant ladite portion de signal, l'indicateur statistique étant associé à l'élément de ladite portion de signal,
- pour chaque élément, comparaison d'au moins un indicateur statistique dudit élément avec des indicateurs statistiques d'autres éléments obtenus pour des portions de signal présentant une même position dans leur suite respective, et en fonction de la comparaison, détermination d'un caractère sain ou non-sain de l'élément.

Dans ce procédé, la pluralité d'éléments comprend P éléments, P étant un entier naturel supérieur à 10, chaque portion de signal étant impliquée dans P-1 couples.

La déformation temporelle dynamique est ici appliquée de façon originale, puisqu'on utilise ensuite un indicateur statistique à partir des distances de couples afin de détecter les portions de signal se distinguant des autres.

Ce procédé est avantageusement complété par les caractéristiques suivantes, prises seules ou en une quelconque de leur combinaison techniquement possible :

- deux portions de signal qui se suivent dans une suite obtenue d'un même signal de mesure se chevauchent partiellement,
- un indicateur statistique est un indicateur de tendance centrale, tel que la moyenne ou la médiane,
- un indicateur statistique est un indicateur de dispersion mesure la variabilité des distances, tel que l'écart-type ou la variance,
- au moins deux indicateurs statistiques sont déterminés, comprenant un indicateur de tendance centrale et un indicateur de dispersion,
- la comparaison d'au moins un indicateur dudit élément avec des indicateurs d'autres éléments comprend une détermination d'une différence entre ledit indicateur statistique et un plus proche autre indicateur statistique d'un autre élément,
- au moins deux indicateurs statistiques sont déterminés, et la comparaison du couple d'indicateurs d'un élément avec des couples d'indicateurs d'autres éléments comprend une modélisation statistique d'une densité de probabilité des valeurs prises par le couple de deux indicateurs statistiques dudit élément,
- chaque portion de signal correspond à une distance de mesure au moins équivalente à une taille typique de défauts recherchés.

L'invention concerne également un produit programme d'ordinateur comprenant des instructions de code de programme pour l'exécution des étapes du procédé selon l'invention, lorsque ledit programme est exécuté sur un ordinateur. Le produit programme d'ordinateur peut prendre la forme d'un médium non volatile sur lequel sont stockées les instructions.

**Présentation des figures**

D'autres caractéristiques, buts et avantages de l'invention ressortiront de la description qui suit, qui est purement illustrative et non limitative, et qui doit être lue en regard des dessins annexés sur lesquels :

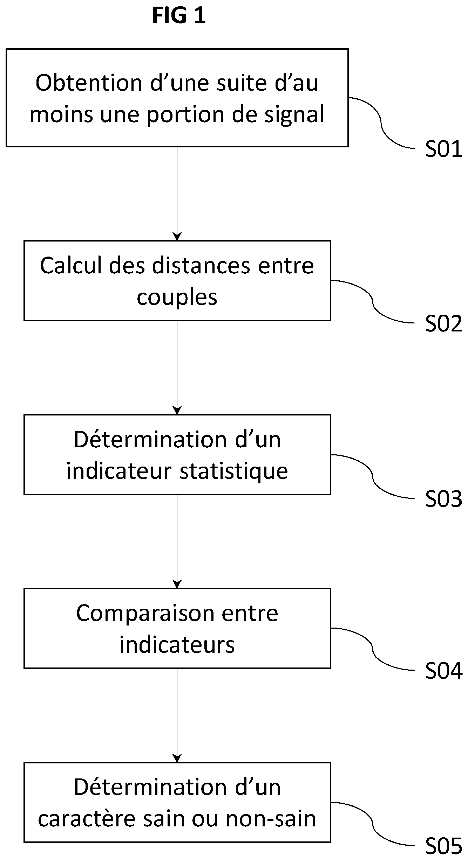

- la figure 1 est un diagramme montrant schématiquement de étapes de mise en œuvre d'un procédé selon un mode de réalisation possible de l'invention ;
- la figure 2 montre un exemple d'alignement par déformation temporelle dynamique entre deux portions de signal d'éléments sains ;
- la figure 3 montre un exemple d'alignement par déformation temporelle dynamique entre une portion de signal d'un élément sain et une portion de signal d'un élément non-sain ;
- la figure 4 montre un exemple d'histogramme de distances de couples pour un élément sain ;
- la figure 5 montre un exemple d'histogramme de distances de couples pour un élément non-sain ;
- la figure 6 montre un exemple de répartition de valeurs pour deux indicateurs statistiques.

**Description détaillée**

L'invention s'applique au contrôle non destructif d'une pluralité d'éléments similaires, dont une majorité est considéré comme présentant un caractère sain. On entend par non-sain un élément présentant un défaut, par exemple mécanique (fissure, corrosion, etc.), qui le rend potentiellement impropre à remplir sa fonction. Par exemple, un organe de fixation tel qu'une vis n'est plus sain s'il est détecté des fissures dans son corps. Les éléments peuvent être de types variés, pourvus qu'on dispose de signaux de mesure correspondant à chacun à un élément différent déterminé. Si le procédé peut être mis en œuvre avec un nombre limité d'éléments, comme par exemple au moins 10 éléments, il est plus fiable et plus avantageux de le mettre en œuvre sur un grand nombre d'éléments. Par conséquent, la pluralité d'éléments comprend de préférence au moins 50 éléments, et de préférence encore au moins 100 éléments.

Les signaux de mesure peuvent être de diverses natures, dès lors que les signaux de mesure sont susceptibles de présenter des variations liées au caractère sain ou non-sain d'un élément inspecté, et en particulier des variations en fonction de la présence de défauts mécaniques sur les éléments inspectés. Par exemple, les signaux de mesure peuvent résulter d'acquisitions ultrasonores, ou des réponses électriques à stimulations telles que la génération de courant de Foucault, la détection d'ondes radio, ou bien encore des images ou vidéos. A titre d'exemple non limitatif, le procédé est décrit dans une application au contrôle d'éléments de fixation mécanique tels que des vis de cloisonnement, et avec des signaux de mesure issus d'acquisitions ultrasonores. Même dans cette application, les signaux de mesure pourraient être différents. Il est à noter que le procédé peut comprendre l'acquisition des signaux de mesure au moyen d'une sonde ou d'un capteur à proximité de chacun des éléments, par exemple successivement d'un élément à l'autre.

Le procédé vise à déterminer le caractère sain ou non-sain de chaque élément. Il est alors possible de poursuivre le procédé avec une inspection plus approfondie des éléments considérés comme non-sains.

Le procédé est mis en œuvre sur un ensemble de signaux de mesure, chacun des signaux de mesure correspondant à un élément différent déterminé. Par exemple, chaque signal de mesure peut être acquis spécifiquement à un élément respectif, typiquement en plaçant une sonde à proximité de l'élément à inspecter : le signal de mesure est alors naturellement associé à un élément particulier. Dans notre exemple, une sonde ultrasonore est placée contre une vis de cloisonnement, puis émet et reçoit des ondes ultrasonores à partir de cette position contre la vis de cloisonnement. Il est également possible que l'acquisition initiale comprenne des signaux de mesure de plusieurs éléments. C'est par exemple le cas lorsqu'une sonde est déplacée le long d'une trajectoire où elle rencontre successivement plusieurs éléments à inspecter. Un découpage du résultat de l'acquisition permet de récupérer des signaux de mesure correspondant chacun à un élément différent déterminé.

De préférence, les signaux de mesure sont de tailles similaires, en termes temporels et/ou spatiaux, ce qui facilite leur manipulation commune dans la suite du procédé.

Une suite d'au moins une portion de signal est obtenue à partir de chaque signal de mesure (étape S1). De préférence, plusieurs portions de signal formant une suite sont extraites de chaque signal de mesure. Si le signal de mesure est issu de la propagation d'une onde, par exemple ultrasonore, ou du déplacement d'une sonde, chaque portion de signal est une fenêtre temporelle correspondant à une distance. Ainsi, chaque portion de signal peut correspondre à une distance de mesure, qui est choisie pour être équivalente à au moins une taille typique de défauts recherchés, et plus avantageusement à environ deux fois une taille typique de défauts recherchés. Typiquement, les défauts recherchés sont de l'ordre du millimètres (fissures, craquelures, etc.), de sorte que chaque portion de signal correspond à une distance de mesure comprise entre 1 mm et 4 mm. Dans l'exemple qui suit, chaque portion de signal correspond à une distance de mesure qui est une profondeur d'environ 2 mm.

De préférence, au moins 2 portions de signal sont extraites d'un même signal de mesure, et de préférence au moins 4 portions de signal sont extraites d'un même signal de mesure. Le nombre de portions de signal dépend cependant bien évidemment de la taille du signal de mesure et de la taille des défauts recherchés. Il est à noter que si le signal de mesure est suffisamment petit, il peut n'être nécessaire de n'extraire qu'une portion de signal, et dans ce cas la suite de portions de signal peut ne comprendre qu'une seule portion de signal. De préférence, les portions de signal de deux éléments différents, présentant une même position dans leur suite respective, font une taille comparable, par exemple en termes de profondeur de mesure. Ainsi, l'extraction de portions de signal peut également servir à s'assurer que les portions de signal comparables portent sur des zones similaires des éléments inspectés, et donc que ces portions de signal d'éléments différents soient comparables.

Par conséquent, même quand les signaux de mesure sont de petites tailles de sorte qu'une seule portion de signal est extraite, l'extraction peut servir au moins à s'assurer que des portions de signal de signaux de mesure différents soient semblables (pas forcément identiques) en termes de représentativité physique de la mesure (par exemple une distance de mesure ou de pénétration). Si les signaux de mesure sont déjà semblables entre eux, l'obtention de la suite d'au moins une portion de signal peut simplement consister en la mise à disposition du signal de mesure.

De préférence, deux portions de signal successives dans une suite d'un même signal de mesure se chevauchent partiellement. En d'autres termes, certaines données du signal de mesure se retrouvent dans ces deux portions de signal successives, ou une partie d'une portion de signal est également dans une autre portion de signal. Le chevauchement avec une autre portion de signal est de préférence compris entre 0,1% et 25% d'une portion de signal, et de préférence entre 0,5% et 10% d'une portion de signal.

Les portions de signal forment une suite en ce sens qu'elles sont ordonnées entres elles : il y a une première portion de signal, suivie par une deuxième portion de signal, suivie par une troisième portion de signal, etc.... On peut noter $a_{i,n}$ la n$^{\text{ième}}$ portion de signal du i$^{\text{ème}}$ signal de mesure. Il est à noter que toutes les portions obtenues d'un signal de mesure ne font pas forcément partie de la suite des portions de mesure, mais que deux portions de signal de la suite conservent le même ordre que dans le signal de mesure : la première portion de signal est dans le signal de mesure avant la seconde portion de signal, la troisième portion de signal est dans le signal de mesure avant la seconde portion de signal, etc.

Ensuite, pour chaque couple associant deux portions de signal présentant une même position dans leur suite respective, une distance entre ces portions de signal dudit couple est calculée : soit $dtw_n(a_{i,n}, a_{j,n})$ la distance entre la $n^{ième}$ portion du $i^{ème}$ signal de mesure et la $n^{ième}$ portion du $j^{ème}$ signal de mesure. Comme évoqué au-dessus, les portions obtenues d'un signal de mesure ne font pas forcément partie de la suite des portions de mesure, et par exemple la suite de portions de mesure peut ne commencer qu'à partir d'une certaine portion extraite, les portions de signal précédentes n'étant alors pas utilisées dans la comparaison et ne faisant donc pas partie de la suite de portion de mesure. De même, il est possibles que les portions de signal de la suite ne soient pas immédiatement consécutives dans le signal de mesure. Cependant, il est constant que l'ordre des portions de signal est respecté : la $(n+1)^{ième}$ portion du $i^{ème}$ signal est comparée avec une portion de mesure du $j^{ème}$ signal de mesure qui est, dans le signal de mesure, après la portion du $j^{ème}$ signal de mesure à laquelle est comparée la $n^{ième}$ portion du $i^{ème}$ signal de mesure.

Comme chaque signal de mesure est associé à un élément particulier, on peut également définir cette distance $dtw_n(a_{i,n}, a_{j,n})$ comme la distance entre la $n^{ième}$ portion du $i^{ème}$ élément et la $n^{ième}$ portion du $j^{ème}$ élément. La distance s'entend ici au sens d'un critère de similarité ou plutôt de dissimilarité (plus la distance est grande, plus la dissimilarité est importante). Il s'agit en particulier de la distance prise en compte dans la déformation temporelle dynamique.

A cet effet, la distance est calculée en utilisant une déformation temporelle dynamique, ou DTW pour l'anglais « Dynamic Time Warping », qui est une méthode déjà employée dans d'autres applications, permettant de mesurer la similarité entre deux suites qui peuvent varier. De façon générale, DTW est une méthode qui recherche un appariement optimal entre deux séries temporelles, sous certaines restrictions. Les séries temporelles sont déformées par transformation non linéaire de la variable temporelle, pour déterminer une mesure de leur similarité, indépendamment de certaines transformations non linéaires du temps.

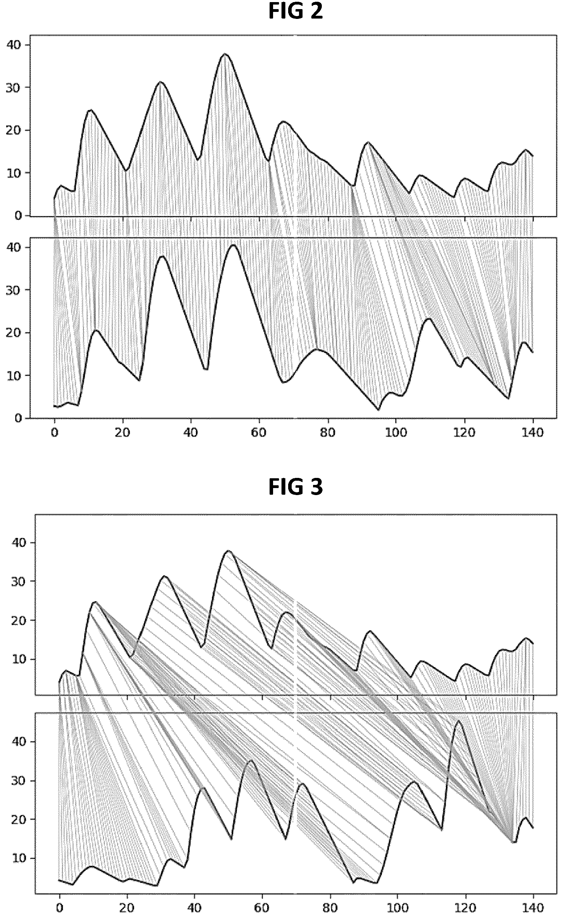

Le DTW définit une distance qui permet de mesurer la similarité entre deux portions $s_1$ et $s_2$ de manière plus pertinente qu'en comparant les signaux point par point, comme c'est par exemple le cas avec la distance euclidienne. Avec DTW, un point de $s_1$ est associé à un ou plusieurs points de $s_2$ (et vice versa), sur la base d'une minimisation d'une fonction de coût. Ceci rend la mesure particulièrement adaptée pour l'analyse de séries temporelles car elle est robuste aux dilatations, aux compressions et aux décalages temporels entre les deux portions de signal. La figure 2 montre un exemple d'alignement par déformation temporelle dynamique entre deux portions de signal d'éléments sains. Les appariements sont illustrés par les traits reliant les points des courbes correspondant aux deux portions de signal. Bien que la plupart des traits soient globalement verticaux, s'approchant d'une distance euclidienne entre points synchrones, certains traits d'appariement sont fortement inclinés, montrant l'influence des transformations subies qui, grâce à la DTW, n'empêchent pas de mettre en évidence une grande similarité.

La figure 3 montre un exemple d'alignement par déformation temporelle dynamique entre une portion de signal d'un élément sain et une portion de signal d'un élément non-sain. Par rapport à la figure 2, on constate des inclinaisons plus importantes des traits d'appariement, et donc des longueurs plus importantes, c'est-à-dire de plus grandes distances d'appariement, indiquant une déformation plus importante à appliquer pour apparier les portions de signal. En outre, on constate que certains points d'une portion de signal sont appariés avec des points de l'autre signal disposés à divers endroits de cet autre signal. Par exemple, plusieurs pics de la portion de signal en haut de la figure 3 sont appariés avec le même point en fin de la portion de signal en bas de la figure 3. Même après alignement, il en résultera une distance finale, mesure de dissimilarité obtenue en calculant les écarts entre les amplitudes des points après appariement, qui restera importante, puisque tous ces pics ne pourront pas être alignés avec le même point de l'autre portion de signal.

Il est cependant difficile de formuler des hypothèses sur la qualité de l'alignement par DTW entre deux portions de signal d'éléments non sains. Il peut arriver que deux portions de signal d'éléments non sains présentent des similitudes, et dans ce cas la mesure DTW pourra être du même ordre de grandeur que la DTW entre deux portions de signal respectives de deux éléments sains. Il peut également arriver que l'alignement entre deux portions de signal respectives d'éléments non-sains soit très compliqué, et dans ce cas la mesure de DTW pourra être du même ordre de grandeur que la DTW entre une portion de signal d'un élément sain et une portion de signal d'un élément non-sain. L'étape préliminaire de découpage du signal en une suite de portions de signal permet d'avoir des ordres de grandeur de DTW locales et comparables. En effet, si on réalisait la mesure de DTW sur tout le signal de mesure, l'impact d'une déformation à un instant ponctuel donné serait trop discret pour être repéré.

La DTW est appliquée à chaque couple associant deux portions de signal présentant une même position dans leur suite respective, de sorte que si la pluralité d'éléments comprend P éléments, P entier naturel supérieur à 10, chaque portion de signal est impliquée dans P-1 couples et il en résulterait P-1 distances impliquant cette portion du signal et donc un élément particulier. De préférence, les distances sont calculées pour plusieurs portions du signal, et de préférence pour toutes les suites de portions de signal.

On peut traduire cette approche par le biais d'une matrice $M_n$ de dimension P x P avec P le nombre d'éléments et donc de signaux de mesure. Les composants $Mn_{i,j}$ de la matrice sont : $Mn_{i,j} = dtw_n(a_{i,n}, a_{j,n})$, avec $dtw_n(a_{i,n}, a_{j,n})$ la distance

entre la n$^{\text{ième}}$ portion du i$^{\text{ème}}$ élément et la n$^{\text{ième}}$ portion du j$^{\text{ième}}$ élément, notée dtw$_n$(a$_i$, a$_j$) pour plus de simplicité :

$$M_n = \begin{pmatrix} dtw_n(a_1, a_1) & dtw_n(a_1, a_2) & \cdots & dtw_n(a_1, a_P) \\ dtw_n(a_2, a_1) & dtw_n(a_2, a_2) & \ldots & dtw_n(a_2, a_P) \\ \vdots & \vdots & \ddots & \vdots \\ dtw_n(a_N, a_1) & dtw_n(a_P, a_2) & \ldots & dtw_n(a_P, a_P) \end{pmatrix}$$

Cette matrice M$_n$ est symétrique, de diagonale nulle, et regroupe toutes les distances de couple possibles pour une même position dans les suites obtenues à partir des signaux de mesure. Il est à noter que la notation matricielle n'est pas indispensable, mais elle permet d'organiser les distances. Ainsi, la colonne j de la matrice M$_n$ regroupe la distance dtw$_n$(a$_j$, a$_j$) entre a$_j$ la n$^{\text{ième}}$ portion de signal de l'élément j et elle-même, qui vaut 0, et l'ensemble des distances dtw$_n$(a$_k$, a$_j$) entre a$_j$ la n$^{\text{ième}}$ portion de signal de l'élément j et les P-1 autres n$^{\text{ième}}$ portions a$_k$ des autres éléments, k≠j.

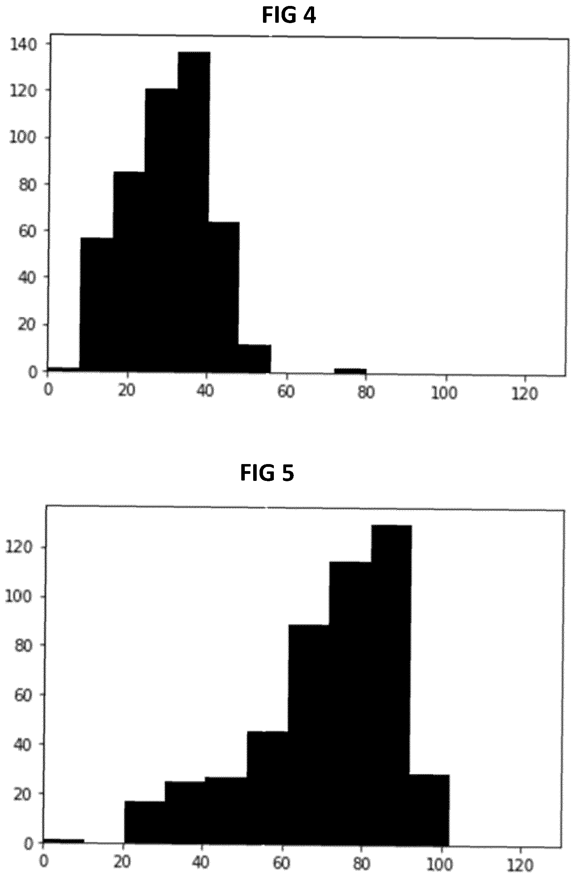

Toutefois, disposer de ces distances de couple ne permet pas de conclure immédiatement au d'un caractère sain ou non-sain d'un élément, puisque qu'il n'existe pas de référence auxquelles les comparer. Il est donc préférable d'adopter une approche statistique visant à identifier les éléments ayant des portions de signal dont les caractéristiques dévient par rapport aux autres. A titre d'illustration, en application de l'inspection de vis de cloisonnement, la figure 4 montre un exemple d'histogramme de distances de couples pour un élément sain, tandis que la figure 5 montre un exemple d'histogramme de distances de couples pour un élément non-sain. En abscisse se trouvent les distances de couples pour cet élément, et en ordonnée le nombre de distances dans des classes de valeurs de distance. Bien entendu, les valeurs indiquées ne sont qu'indicatives, celles-ci dépendant de nombreux paramètres tels que les amplitudes des signaux de mesure. On remarque que l'histogramme de distances de couples pour un élément non-sain est généralement translaté et dilaté par rapport à l'histogramme de distances de couples pour un élément sain. Ainsi, une approche statistique peut permettre d'identifier de telles déviations.

Par conséquent, pour chaque portion de signal, au moins un indicateur statistique est déterminé à partir des distances de couples impliquant ladite portion de signal (étape S03), l'indicateur statistique étant associé à l'élément de ladite portion de signal. En reprenant la notation matricielle, les distances de couples impliquant un élément j, et donc impliquant la portion de signal a$_j$, correspond à la colonne j de la matrice M$_n$.

De préférence, un indicateur statistique est un indicateur de tendance centrale, tel que la moyenne ou la médiane, ou un indicateur statistique est un indicateur de dispersion mesure la variabilité des distances, tel que l'écart-type ou la variance. De préférence, au moins deux indicateurs statistiques sont déterminés, comprenant un indicateur de tendance centrale et un indicateur de dispersion. Typiquement, les indicateurs statistiques sont la moyenne μ et l'écart-type σ. Il est à noter que le ou les indicateurs statistiques peuvent être déterminés directement à partir des distances de couples, ou bien à partir d'un histogramme tel que celui illustré dans les figures 4 et 5.

Pour chaque élément, il est ensuite procédé à une comparaison d'au moins un indicateur dudit élément avec des indicateurs d'autres éléments (étape S04). De préférence, au moins deux indicateurs statistiques sont déterminés, et la comparaison est effectuée par couples d'indicateur, un couple d'indicateurs d'un élément étant comparés avec des couples d'indicateurs d'autres éléments. En fonction de la comparaison, le caractère sain ou non-sain d'un élément est déterminé (étape S05), de préférence sur la base d'une déviation des indicateurs statistique par rapport aux autres.

La comparaison vise à identifier les éléments pour lesquels le ou les indicateurs statistiques sont éloignés de ceux des éléments sains, supposés majoritaires. Plusieurs approches peuvent être adoptées. La comparaison de l'au moins un indicateur dudit élément avec des indicateurs d'autres éléments peut comprendre une détermination d'une différence entre ledit indicateur statistique et un plus proche autre indicateur statistique d'un autre élément, et même entre ledit indicateur statistique et plusieurs plus proches autres indicateurs statistiques d'autres éléments. Par exemple, la méthode des « K plus proches voisins », consiste, pour chaque élément, à calculer la distance euclidienne à son K-ème voisin le plus proche. Un seuil est ensuite choisi sur cette distance pour décider si l'élément est potentiellement non-sain. D'autres variantes peuvent être utilisées, comme par exemple la méthode du facteur d'exception locale ou "local outlier factor" en anglais.

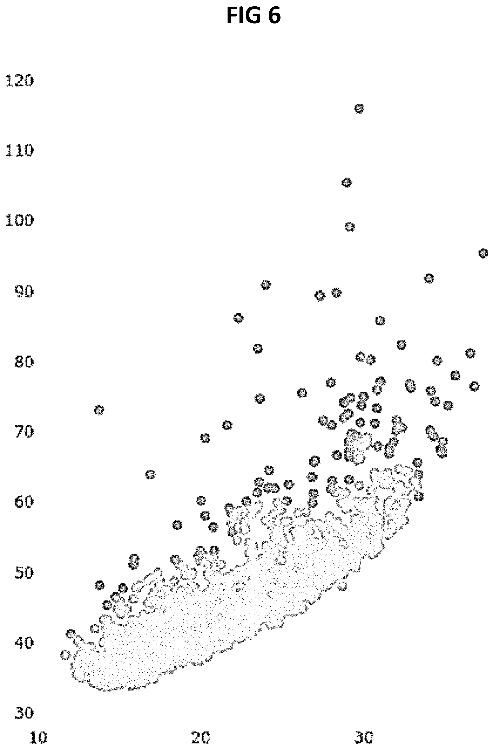

La figure 6 montre un exemple de représentation spatiale en deux dimensions de couples {moyenne, écart-type} pour des distances entre des vis de cloisonnement. En ordonnée figurent les moyennes et en abscisses figurent les écart-types. En appliquant la méthode des « K plus proches voisins », des éléments dont les indicateurs statistiques déviaient de la majorité ont été identifiés, et apparaissent en noir sur la figure 6, tandis que la majorité des éléments dont les indicateurs statistiques étaient proches les uns des autres apparaissent en blanc, et qui sont donc considérés comme sains.

Tout d'abord, on constate que des d'éléments sains présentent des moyennes et des écart-types supérieurs à ceux d'éléments non-sains, et que certains éléments non-sains présentent des moyennes bien supérieures à celles des éléments sains, notamment supérieures à 70. Ainsi, une simple comparaison de valeurs avec un seuil fixe permettrait de détecter certains éléments non-sains, mais déterminerait également un caractère sain pour beaucoup d'éléments non-sains. Une telle approche n'est donc pas totalement satisfaisante. Au contraire, identifier les éléments dont les indicateurs

devient de ceux de la majorité des autres permet de détecter des éléments potentiellement non-sains même lorsque ceux-ci présentent des moyennes et des écart-types inférieures aux moyennes et écart-types d'éléments sains.

Alternativement, la comparaison du couple d'indicateurs d'un élément avec des couples d'indicateurs d'autres éléments comprend une modélisation statistique d'une densité de probabilité des valeurs prises le couple de deux indicateurs statistiques dudit élément. Il peut être utilisé par exemple des méthodes paramétriques (Gaussiennes, mélange de Gaussiennes) ou bien des méthodes non paramétriques, comme par exemple une méthode à noyaux.

Il est à noter qu'il y a au moins un indicateur statistique par portion de signal, de sorte que la comparaison peut porter sur plusieurs indicateurs statistiques de plusieurs portions de signal d'un même élément. Il est alors par exemple possible de déterminer un caractère non-sain s'il apparaît que l'indicateur statistique diffère des autres pour seule position dans la suite, ou au contraire requérir que l'indicateur statistique doive différer des autres dans plusieurs positions dans la suite pour que l'élément soit considéré comme sain. Afin de ne pas rater d'élément potentiellement défectueux, il est préférable de considérer comme non-sain un élément dont le ou les indicateurs statistiques diffèrent des autres pour au moins une position dans la suite de portions de signal.

Une fois déterminée le caractère sain ou non-sain de chacun des éléments, il est possible de mettre en œuvre une inspection approfondie des éléments non-sains, ou directement une intervention physique sur ceux-ci, tels que leur remplacement ou leur mise au rebut. A cet égard, il est à noter que le procédé est configuré pour être capable de détecter tous les éléments non-sains, et qu'il est moins dommageable de considérer comme non-sain un élément sain plutôt que de prendre le risque d'estimer sain un élément qui n'est pas sain.

L'invention n'est pas limitée au mode de réalisation décrit et représenté aux figures annexées. Des modifications restent possibles sans sortir pour autant du domaine de protection de l'invention, qui est défini par les revendications suivantes.

**Revendications**

1. Procédé de contrôle non destructif d'une pluralité d'éléments similaires à partir de signaux de mesure, chacun des signaux de mesure correspondant à un élément différent déterminé, comprenant les étapes suivantes :

   - obtention (S01) d'une suite d'au moins une portion de signal à partir de chaque signal de mesure,
   - pour chaque couple associant deux portions de signal présentant une même position dans leur suite respective, une distance (S02) entre ces portions de signal dudit couple est calculée, ladite distance étant calculée en utilisant une déformation temporelle dynamique,
   - pour chaque portion de signal, détermination (S03) d'au moins un indicateur statistique à partir des distances de couples impliquant ladite portion de signal, l'indicateur statistique étant associé à l'élément de ladite portion de signal,
   - pour chaque élément, comparaison (S04) d'au moins un indicateur statistique dudit élément avec des indicateurs statistiques d'autres éléments obtenus pour des portions de signal présentant une même position dans leur suite respective, et en fonction de la comparaison, détermination (S05) d'un caractère sain ou non-sain de l'élément,

   et dans lequel la pluralité d'éléments comprend P éléments, P étant un entier naturel supérieur à 10, chaque portion de signal étant impliquée dans P-1 couples.

2. Procédé selon la revendication 1, dans lequel deux portions de signal qui se suivent dans une suite obtenue d'un même signal de mesure se chevauchent partiellement.

3. Procédé selon l'une quelconque des revendications précédentes, dans lequel un indicateur statistique est un indicateur de tendance centrale, tel que la moyenne ou la médiane.

4. Procédé selon l'une quelconque des revendications précédentes, dans lequel un indicateur statistique est un indicateur de dispersion mesure la variabilité des distances, tel que l'écart-type ou la variance.

5. Procédé selon l'une quelconque des revendications précédentes, dans lequel au moins deux indicateurs statistiques sont déterminés, comprenant un indicateur de tendance centrale et un indicateur de dispersion.

6. Procédé selon l'une quelconque des revendications précédentes, dans lequel la comparaison d'au moins un indicateur dudit élément avec des indicateurs d'autres éléments comprend une détermination d'une différence entre ledit indicateur statistique et un plus proche autre indicateur statistique d'un autre élément.

7. Procédé selon l'une quelconque des revendications 1 à 6, dans lequel au moins deux indicateurs statistiques sont déterminés, et la comparaison du couple d'indicateurs d'un élément avec des couples d'indicateurs d'autres éléments comprend une modélisation statistique d'une densité de probabilité des valeurs prises par le couple de deux indicateurs statistiques dudit élément.

8. Procédé selon l'une quelconque des revendications précédentes, dans lequel chaque portion de signal correspond à une distance de mesure au moins équivalente à une taille typique de défauts recherchés.

9. Produit programme d'ordinateur comprenant des instructions de code de programme pour l'exécution des étapes du procédé selon l'une quelconque des revendications précédentes, lorsque ledit programme est exécuté sur un ordinateur.

**Patentansprüche**

1. Verfahren zum zerstörungsfreien Überprüfen einer Vielzahl von ähnlichen Elementen ausgehend von Messsignalen, wobei jedes der Messsignale einem bestimmten unterschiedlichen Element entspricht, umfassend folgende Schritte:

   - Erzielen (S01) einer Reihe von mindestens einem Signalabschnitt aus jedem Messsignal,
   - für jedes Paar, das zwei Signalabschnitte verknüpft, welche die gleiche Position in ihrer jeweiligen Reihe aufweisen, Berechnen (S02) eines Abstands zwischen diesen Signalabschnitten des Paars, wobei der Abstand unter Verwendung einer dynamischen zeitlichen Verzerrung berechnet wird,
   - für jeden Signalabschnitt, Bestimmen (S03) mindestens eines statistischen Indikators aus den Abständen von Paaren, die den Signalabschnitt einbeziehen, wobei der statistische Indikator mit dem Element des Signalabschnitts verknüpft ist,
   - für jedes Element, Vergleichen (S04) mindestens eines statistischen Indikators des Elements mit statistischen Indikatoren anderer Elemente, die für Signalabschnitte erzielt werden, welche die gleiche Position in ihrer jeweiligen Reihe aufweisen, und in Abhängigkeit von dem Vergleich, Bestimmen (S05) eines gesunden oder nicht gesunden Merkmals des Elements,

   und wobei die Vielzahl von Elementen P Elemente umfasst, wobei P eine natürliche Ganzzahl größer als 10 ist, wobei jeder Signalabschnitt in P-1 Paaren einbezogen ist.

2. Verfahren nach Anspruch 1, wobei sich zwei Signalabschnitte, die in einer Reihe, die aus dem gleichen Messsignal erzielt wird, aufeinander folgen, teilweise überlappen.

3. Verfahren nach einem beliebigen der vorhergehenden Ansprüche, wobei ein statistischer Indikator ein Zentraltendenzindikator, wie etwa eines Mittel- oder Medianwertes, ist.

4. Verfahren nach einem beliebigen der vorhergehenden Ansprüche, wobei ein statistischer Indikator ein Streuungsindikator ist, der die Variabilität der Abstände, wie etwa die Standardabweichung oder die Varianz, misst.

5. Verfahren nach einem beliebigen der vorhergehenden Ansprüche, wobei mindestens zwei statistische Indikatoren bestimmt werden, die einen Zentraltendenzindikator und einen Streuungsindikator umfassen.

6. Verfahren nach einem beliebigen der vorhergehenden Ansprüche, wobei der Vergleich mindestens eines Indikators des Elements mit Indikatoren anderer Elemente eine Bestimmung einer Differenz zwischen dem statistischen Indikator und einem anderen näheren statistischen Indikator eines anderen Elements umfasst.

7. Verfahren nach einem beliebigen der Ansprüche 1 bis 6, wobei mindestens zwei statistische Indikatoren bestimmt werden, und der Vergleich des Indikatorenpaars eines Elements mit Indikatorenpaaren anderer Elemente eine statistische Modellierung einer Wahrscheinlichkeitsdichte der Werte, die von dem Paar von zwei statistischen Indikatoren des Elements übernommen werden, umfasst.

8. Verfahren nach einem beliebigen der vorhergehenden Ansprüche, wobei jeder Signalabschnitt einem Messabstand entspricht, der mit einer typischen Größe gesuchter Defekte mindestens vergleichbar ist.

9. Computerprogrammprodukt, umfassend Programmcodeanweisungen zur Ausführung der Schritte des Verfahrens nach einem beliebigen der vorhergehenden Ansprüche, wenn das Programm auf einem Computer ausgeführt wird.

**Claims**

1. A method for non-destructive testing of a plurality of components based on measurement signals, each of the measurement signals corresponding to a predetermined different component, comprising the following steps:

   - obtaining (S01) a sequence of at least one signal portion based on each measurement signal,
   - for each pair associating two signal portions having one and the same position in their respective sequence, a distance (S02) between these signal portions of said pair is computed, said distance being computed using dynamic time warping,
   - for each signal portion, determining (S03) at least one statistical indicator based on the distances of pairs involving said signal portion, the statistical indicator being associated with the component of said signal portion,
   - for each component, comparing (S04) at least one statistical indicator of said component to statistical indicators of other components, which are obtained for signal portions having one and the same position in their respective sequence, and as a function of the comparison, determining (S05) the soundness or unsoundness of the component,

   wherein the plurality of components comprises P components, P being a natural integer greater than 10, and each signal portion is involved in P-1 pairs.

2. The method as claimed in claim 1, wherein two signal portions that follow one another in a sequence obtained from one and the same measurement signal partially overlap.

3. The method as claimed in any one of the preceding claims, wherein a statistical indicator is a central tendency indicator, such as the mean or the median.

4. The method as claimed in any one of the preceding claims, wherein a statistical indicator is a dispersion indicator measuring the variability of the distances, such as the standard deviation or the variance.

5. The method as claimed in any one of the preceding claims, wherein at least two statistical indicators are determined, comprising a central tendency indicator and a dispersion indicator.

6. The method as claimed in any one of the preceding claims, wherein the comparison of at least one indicator of said component to indicators of other components comprises the determination of a difference between said statistical indicator and another nearer statistical indicator of another component.

7. The method as claimed in any one of claims 1 to 6, wherein at least two statistical indicators are determined, and the comparison of the indicator pair of one component to indicator pairs of other components comprises a statistical modelling of a probability density of the values taken by the pair of two statistical indicators of said component.

8. The method as claimed in any one of the preceding claims, wherein each signal portion corresponds to a measurement distance at least equivalent to a typical size of sought defects.

9. A computer program product comprising program code instructions for executing the steps of the method as claimed in any one of the preceding claims, when said program is executed on a computer.

**FIG 1**

Obtention d'une suite d'au moins une portion de signal — S01

Calcul des distances entre couples — S02

Détermination d'un indicateur statistique — S03

Comparaison entre indicateurs — S04

Détermination d'un caractère sain ou non-sain — S05

## FIG 2

## FIG 3

**FIG 4**

**FIG 5**

# FIG 6

## RÉFÉRENCES CITÉES DANS LA DESCRIPTION

**Documents brevets cités dans la description**

- FR 3029288

**Littérature non-brevet citée dans la description**

- **VAN VAERENBERGH et al.** Pattern Localization in Time Series Through Signal-To-Model Alignment in Latent Space. *Conference: ICASSP 2018 - IEEE International Conference on Acoustics, Speech and Signal Processing*, February 2018

- A general anomaly detection framework for fleet-based condition monitoring of machines. **HENDRICKX K et al.** Mecahnical Systems and Signal Processing. Elsevier, 11 January 2020, vol. 139